# EUROPEAN PATENT APPLICATION

(11) **EP 4 379 058 A1**
(43) Date of publication of application: **05.06.2024**
(21) Application number: 21952033.5
(22) Date of filing: 07.12.2021
(51) Int. Cl.: C12N 15/79, C07K 14/405, C12N 15/87

(54) **BIOMINERALIZATION VECTOR AND TRANSFORMANT FOR TRANSFORMATION OF CHLORELLA VULGARIS**

(30) Priority: 26.07.2021 KR 20210097954
(71) Applicant: Green Mineral Inc., Seoul 04147 (KR)
(72) Inventor: JUNG, Kwang Hwan, Seongnam-si, Gyeonggi-do 13566 (KR); SHIM, Jin Gon, Seoul 07201 (KR); LEE, Hosuk Sean, Seoul 03113 (KR)
(74) Representative: Plougmann Vingtoft a/s
(86) International application number: PCT/KR2021/018446
(87) International publication number: WO 2023/008664

(57) **Abstract**

The present invention relates to a vector system for transformation of *Chlorella vulgaris,* a *Chlorella vulgaris* transformation method using same, and a *Chlorella vulgaris* transformant.

## Description

### Technical Field

This patent application claims priority to and the benefit of Korean Patent Application No. 10-2021-0097954 filed with the Korean Intellectual Property Office on July 26, 2021, the disclosure of which is incorporated herein by reference.

The present disclosure relates to a vector system for the transformation of *Chlorella vulgaris,* a method for transforming *Chlorella vulgaris* using same, and a *Chlorella vulgaris* transformant.

### Background Art

*Chlorella vulgaris* is a unicellular microalga in the Chlorophyta phylum. *Chlorella vulgaris,* possessing a photosynthetic apparatus, can grow rapidly using only light, carbon dioxide, water, and a small amount of minerals through photosynthesis and thus is widely used in research for mass production of useful substances (photobioreactors).

Additionally, with a lipid content of about 42% of its biomass, *Chlorella vulgaris* is also extensively used in research for biofuel development as a potential alternative to biodiesel. Recent studies have shown that *Chlorella vulgaris* can be used to remove the radioactive isotope ⁹⁰Sr.

Selectable markers such as the hygromycin B resistance gene, zeocin resistance gene, and chloramphenicol acetyltransferase gene (CAT gene) have been used in electroporation, resulting in integration into chromosomal DNA. However, the transformants were only temporary, losing their resistance after a short period.

Transformation methods include the use of glass beads, electroporation, and Agrobacterium-mediated transformation. However, due to the lack of efficient selectable markers and transformation systems, the use of *Chlorella vulgaris* remains limited.

As technology advances, the consumption of electricity is rapidly increasing, and the efficiency of energy consumption and production is improving. The world is focusing on nuclear power for efficient production of electricity. However, issues of nuclear accidents and contamination of water with radioactivity and metal ions from power plants remain unresolved problems.

Furthermore, the dependence on imports of rare metals like Sr, Cs, Li, which are used in industrial settings, poses a national challenge. With the growing market for secondary batteries, these problems are becoming more pronounced, and recycling them presents significant industrial and national issues.

### Disclosure of Invention

### Technical Problem

In pursuit of developing a transformation method for *Chlorella vulgaris,* the present inventors, after thorough and intensive research, predicted and obtained the sequences of the promoter and terminator regions of *Coccomyxa* C-169 (hereinafter referred to as C-169; previously known as *Chlorella vulgaris* but renamed) and fused the sequences with the *Sh ble* gene from *Streptomyces verticillus* bleomycin (*Sh ble*)*.* No introns were inserted in the middle of the *Sh ble* gene, and the synthesized DNA fragment was treated with SwaI/KpnI restriction enzymes and cloned into the pSP124S vector, which was then named pKA650.

Furthermore, the pKa650 vector was cloned with the beta-type carbonic anhydrase gene as the target gene in the middle of the *Streptomyces verticillus* bleomycin (*Sh ble*) gene and was named pJG002.

Subsequently, after transformation via gold particle bombardment, the cells were cultured in a medium containing zeocin for selective selection. The transformation was completed by obtaining colonies through selection, thus producing transformants.

Therefore, an aspect of the present disclosure is to provide a vector system for the transformation of *Chlorella vulgaris.*

Also, another aspect of the present disclosure is to provide a method for the transformation of *Chlorella vulgaris.*

Furthermore, a further aspect of the present disclosure is to provide a *Chlorella vulgaris* transformant.

### Solution to Problem

The present disclosure pertains to a vector system for the transformation of *Chlorella vulgaris,* a method for transforming *Chlorella vulgaris,* using same, and a microalga transformant.

Below, a detailed description will be given of the present disclosure.

An aspect of the present disclosure contemplates a vector system for the transformation of *Chlorella vulgaris,* comprising:
a promoter for *Coccomyxa* C-169 ribulose-1,5-bisphosphate carboxylase/oxygenase (Rubisco) small subunit 2 (rbcS2) gene;
a nucleotide sequence coding for a target protein; and
a terminator sequence of *Coccomyxa* C-169 rbcS2 gene.

In the present disclosure, the promoter may include the nucleotide sequence of SEQ ID NO: 2, or a nucleotide sequence substantially identical to that of SEQ ID NO: 2, for example, consisting of the nucleotide sequence of SEQ ID NO: 2, but with no limitations thereto.

In an embodiment of the present disclosure, the promoter including the nucleotide sequence of SEQ ID NO: 2, which corresponds to a part of the nucleotide sequence of SEQ ID NO: 1, may additionally contain 1 to 50 base pairs (bp) at the 3' end of the sequence of SEQ ID NO: 2, but with no limitations thereto.

The terminator sequence in the present disclosure may include the nucleotide sequence of SEQ ID NO: 3, or a nucleotide sequence substantially identical to that of SEQ ID NO: 3, for example, consisting of the nucleotide sequence of SEQ ID NO: 3, but with no limitations thereto.

The target protein-encoding nucleotide sequence in the present disclosure may be operatively linked to the promoter.

As used herein, the term "operatively linked" refers to a functional connection between a nucleic acid expression regulatory sequence (e.g., promoter sequence, signal sequence, or array of transcription factor binding sites) and another nucleic acid sequence, whereby the regulatory sequence regulates transcription and/or translation of the other nucleic acid sequence.

In the present disclosure, the target protein-encoding nucleotide sequence may be linked to the 3' end of the terminator sequence, but with no limitations thereto.

The vector system in the present disclosure may additionally include a selectable marker.

The selectable marker in the present disclosure may be an antibiotic resistance gene, but is not limited thereto.

In the present disclosure, the antibiotic may be one or more selected from the group consisting of spectinomycin, paromomycin, ampicillin, zeocin, and bleomycin, but is not limited thereto.

In the present disclosure, Selectable markers can include various selectable marker genes for the desired antibiotic. Examples include aminoglycoside phosphotransferase, which confers kanamycin resistance, and chloramphenicol acetyltransferase, which is involved in chloramphenicol resistance.

As for a selectable marker for bleomycin, its nucleotide sequence may include the nucleotide sequence of SEQ ID NO: 4, or a nucleotide sequence substantially identical to SEQ ID NO: 4, for example, consisting of the nucleotide sequence of SEQ ID NO: 4, but is not limited thereto.

In the present disclosure, the method for selecting selectable marker-introduced, transformed *Chlorella vulgaris* may be easily carried out using the phenotype expressed by the selectable marker in a manner well known in the art. For example, if the selectable marker is a specific antibiotic resistance gene, the transformant can be easily selected by culturing in a medium containing the antibiotic.

The vector system in the present disclosure may additionally include a gene coding for a reporter molecule.

The reporter molecule in the present disclosure may be one or more selected from the group consisting of growth-promoting proteins, fluorescent proteins, and hydrolases, but is not limited thereto.

The fluorescent protein in the present disclosure may be luciferase, but is not limited thereto.

The hydrolase in the present disclosure may be β-glucuronidase, but is not limited thereto.

The vector system in the present disclosure may include the nucleotide sequence of SEQ ID NO: 1, or a nucleotide sequence substantially identical to SEQ ID NO: 1, for example, consisting of the nucleotide sequence of SEQ ID NO: 1, but with no limitations thereto.

The vector system in the present disclosure may be for transformation of *Chlorella vulgaris* using gold particle bombardment.

As used herein, the term "vector" refers to a means for expressing a target gene in a host cell. Examples include plasmid vectors, cosmid vectors, bacteriophage vectors, and viral vectors such as adenovirus vectors, retrovirus vectors, and adeno-associated virus vectors.

Vectors available as recombinant vectors in the present disclosure may be constructed by manipulating plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), phages (e.g., λgt4λB, λ-Charon, λΔz1, and M13), or viruses (e.g., SV40). For example, it may be based on the pSP124S vector backbone, but with no limitations thereto.

In the present disclosure, the vector system can typically be constructed as a cloning vector or as an expression vector.

For expression vectors in the present disclosure, conventional vectors used in the industry for expressing target proteins in plants, animals, or microorganisms can be used.

The vector system in the present disclosure may be constructed through various methods well known in the art.

As used herein, the term "substantial identity" means that two sequences are aligned to correspond to each other as much as possible and show a homology of 70% or greater, 90% or greater, or 98% or greater therebetween as analyzed.

Another aspect of the present disclosure pertains to a method for producing a *Chlorella vulgaris* transformant, the method comprising a transforming step of:
introducing into *Chlorella vulgaris* a vector carrying a promoter for *Coccomyxa* C-169 rbcS2 gene, a target protein-encoding nucleotide sequence, and a terminator sequence of *Coccomyxa* C-169 rbcS2 gene.

In the present disclosure, the transformation step may be performed using gold particle bombardment.

In the present disclosure, the cells used for the transformation step may be in a log phase. The most efficient transformation can be obtained in the log phase.

In the present disclosure, the log phase may be when the OD₆₈₆ value is between 0.4 and 0.6, between 0.45 and 0.6, between 0.5 and 0.6, or between 0.55 and 0.6, for example, 0.6.

In the present disclosure, the cells in the transformation step may have a density of 5.0*10⁶ to 8.0*10⁷ per 60mm diameter, 1.0*10⁷ to 8.0*10⁷ per 60mm diameter, 2.0*10⁷ to 8.0*10⁷ per 60mm diameter, 3.0*10⁷ to 8.0*10⁷ per 60mm diameter, 4.0*10⁷ to 8.0*10⁷ per 60mm diameter, 1.0*10⁷ to 7.0*10⁷ per 60mm diameter, 2.0*10⁷ to 7.0*10⁷ per 60mm diameter, 3.0*10⁷ to 7.0*10⁷ per 60mm diameter, 4.0*10⁷ to 7.0*10⁷ per 60mm diameter, 1.0*10⁷ to 6.0*10⁷ per 60mm diameter, 2.0*10⁷ to 6.0*10⁷ per 60mm diameter, 3.0*10⁷ to 6.0*10⁷ per 60mm diameter, 4.0*10⁷ to 6.0*10⁷ per 60mm diameter, 1.0*10⁷ to 5.0*10⁷ per 60mm diameter, 2.0*10⁷ to 5.0*10⁷ per 60mm diameter, 3.0*10⁷ to 5.0*10⁷ per 60mm diameter, or 4.0*10⁷ to 5.0*10⁷ per 60mm diameter, for example, 4.8*10⁷ per 60mm diameter.

In the present disclosure, the transformation step may be carried out under a vacuum of 27.0 to 29.0 inches Hg, 27.5 to 29.0 inches Hg, 28.0 to 29.0 inches Hg, 28.5 to 29.0 inches Hg, for example, 29.0 inches Hg.

In the present disclosure, the target distance in the transformation step may be 3 to 9, for example, 3, 6, or 9.

In the present disclosure, the transformation step may be carried out at a pressure of 1200 to 1300 psi, 1210 to1300 psi, 1220 to1300 psi, 1230 to 1300 psi, 1240 to 1300 psi, 1250 to 1300 psi, 1260 to 1300 psi, 1270 to 1300 psi, 1280 to 1300 psi, 1290 to 1300 psi, for example, 1300 psi.

In the present disclosure, the promoter may include the nucleotide sequence of SEQ ID NO: 2, or a nucleotide sequence substantially identical to SEQ ID NO: 2, for example, consisting of the nucleotide sequence of SEQ ID NO: 2, but is not limited thereto.

In an embodiment of the present disclosure, the promoter including the nucleotide sequence of SEQ ID NO: 2, which corresponds to a part of the nucleotide sequence of SEQ ID NO: 1, may additionally contain 1 to 50 base pairs (bp) at the 3' end of the sequence of SEQ ID NO: 2, but with no limitations thereto.

The terminator sequence in the present disclosure may include the nucleotide sequence of SEQ ID NO: 3, or a nucleotide sequence substantially identical to SEQ ID NO: 3, for example, consisting of the nucleotide sequence of SEQ ID NO: 3, but is not limited thereto.

In the present disclosure, the target protein-encoding nucleotide sequence may be operatively linked to the promoter.

The target protein-encoding nucleotide sequence in the present disclosure may be linked at the 3' end of the terminator sequence, but with no limitations thereto.

The vector in the present disclosure may additionally include a selectable marker.

In the present disclosure, the selectable marker may be an antibiotic resistance gene, but is not limited thereto.

The antibiotic may be one or more selected from a group consisting of spectinomycin, paromomycin, ampicillin, zeocin, and bleomycin, but is not limited thereto.

The selectable marker may be selected from various selectable marker genes for conventional desired antibiotics. Examples include aminoglycoside phosphotransferase, which confers kanamycin resistance, and chloramphenicol acetyltransferase, which is involved in chloramphenicol resistance.

In the present disclosure, the method for selecting selectable marker-introduced, transformed *Chlorella vulgaris* may be easily performed using the phenotype expressed by the selectable marker in a manner known in the art. For example, if the selectable marker is a specific antibiotic resistance gene, the transformant may be easily selected by culturing in a medium containing the antibiotic.

The vector system in the present disclosure may additionally include a gene coding for a reporter molecule.

The reporter molecule may be one or more selected from the group consisting of growth-promoting proteins, fluorescent proteins, and hydrolases, but is not limited thereto.

The fluorescent protein in the present disclosure may be luciferase, but is not limited thereto.

The hydrolase in the present disclosure may be β-glucuronidase, but is not limited thereto.

The vector in the present disclosure may include the nucleotide sequence of SEQ ID NO: 1, or a nucleotide sequence substantially identical to SEQ ID NO: 1, for example, consisting of the nucleotide sequence of SEQ ID NO: 1, but with no limitations thereto.

The method for constructing a transformant in the present disclosure provides a *Chlorella vulgaris* transformant.

Another aspect of the present disclosure pertains to a biomineralization vector for the transformation of *Chlorella vulgaris* using the vector system and a transformant having same introduced thereinto.

An aspect of the present disclosure relates to a biomineralization vector for the transformation of *Chlorella vulgaris,* the vector carrying:
a promoter for *Coccomyxa* C-169 ribulose-1,5-bisphosphate carboxylase/oxygenase (RuBisCo) small subunit 2 (rbcS2) gene;
a nucleotide sequence coding for beta-type carbonic anhydrase from *Coccomyxa* subellipsoidea C-169; and
a terminator sequence of the *Coccomyxa* C-169 rbcS2 gene.

In this invention, the promoter may include the nucleotide sequence of SEQ ID NO: 2, or a nucleotide sequence substantially identical to SEQ ID NO: 2, for example, consisting of the nucleotide sequence of SEQ ID NO: 2, but is not limited thereto.

In an embodiment of the present disclosure, the promoter including the nucleotide sequence of SEQ ID NO: 2, which corresponds to a part of the nucleotide sequence of SEQ ID NO: 7, may additionally contain 1 to 50 base pairs (bp) at the 3' end of the sequence of SEQ ID NO: 2, but with no limitations thereto.

The terminator sequence in the present disclosure may include the nucleotide sequence of SEQ ID NO: 3, or a nucleotide sequence substantially identical to SEQ ID NO: 3, for example, consisting of the nucleotide sequence of SEQ ID NO: 3, but is not limited thereto.

In the present disclosure, the nucleotide sequence coding for beta-type carbonic anhydrase from *Coccomyxa* subellipsoidea C-169 may be operatively linked to the promoter.

As used herein, the term "operatively linked" refers to a functional connection between a nucleic acid expression regulatory sequence (e.g., promoter sequence, signal sequence, or array of transcription regulatory factor binding sites) and another nucleic acid sequence, whereby the regulatory sequence regulates transcription and/or translation of the other nucleic acid sequence.

The nucleotide sequence coding for beta-type carbonic anhydrase from *Coccomyxa* subellipsoidea C-169 in the present disclosure may be linked at the 3' end of the terminator sequence, but with no limitations thereto.

In the present disclosure, the vector may additionally include a selectable marker.

In the present disclosure, the selectable marker may be an antibiotic resistance gene, but is not limited thereto.

The antibiotic in the present disclosure may be one or more selected from a group consisting of spectinomycin, paromomycin, ampicillin, zeocin, and bleomycin, but is not limited thereto.

The selectable marker may be selected from various selectable marker genes for conventional desired antibiotics. Examples include aminoglycoside phosphotransferase, which confers kanamycin resistance, and chloramphenicol acetyltransferase, which is involved in chloramphenicol resistance.

If the selectable marker is bleomycin, the marker may include the nucleotide sequence of SEQ ID NO: 4, or a nucleotide sequence substantially identical to SEQ ID NO: 4, for example, consisting of the nucleotide sequence of SEQ ID NO: 4, but is not limited thereto.

The method of selecting selectable marker-introduced, transformed *Chlorella vulgaris* in the present disclosure may be easily performed using the phenotype expressed by the selectable marker in a manner known in the art. For example, if the selectable marker is a specific antibiotic resistance gene, the transformant can be easily selected by culturing in a medium containing the antibiotic.

The vector in the present disclosure may additionally include a gene coding for a reporter molecule.

The reporter molecule in the present disclosure may be one or more selected from the group consisting of growth-promoting proteins, fluorescent proteins, and hydrolases, but is not limited thereto.

The fluorescent protein in the present disclosure may be luciferase, but is not limited thereto.

The hydrolase in the present disclosure may be β-glucuronidase, but is not limited thereto.

The vector in the present disclosure may include the nucleotide sequence of SEQ ID NO: 7, or a nucleotide sequence substantially identical to SEQ ID NO: 7, for example, consisting of the nucleotide sequence of SEQ ID NO: 7, but is not limited thereto.

Another aspect of the present disclosure pertains to a transformant transformed with a biomineralization vector for the transformation of *Chlorella vulgaris,* the vector comprising:
a promoter for *Coccomyxa* C-169 ribulose-1,5-bisphosphate carboxylase/oxygenase (RuBisCo) small subunit 2 (rbcS2) gene;
a nucleotide sequence coding for beta-type carbonic anhydrase from *Coccomyxa* subellipsoidea C-169; and
a terminator sequence of the *Coccomyxa* C-169 rbcS2 gene.

To avoid the complexity of the description, the content of the biomineralization vector for transforming *Chlorella vulgaris* is omitted, as it is the same as stated in the foregoing.

Another aspect of the present disclosure relates to a method for producing a target protein, comprising the steps of:
introducing into *Chlorella vulgaris* a vector including a promoter for *Coccomyxa* C-169 rbcS2 gene, a nucleotide sequence coding for a target protein, and a terminator sequence of *Coccomyxa* C-169 rbcS2 gene;
cultivating the transformed *Chlorella vulgaris* to obtain the target protein.

The target protein-coding nucleotide sequence in the present disclosure may be operatively linked to the promoter, but with no limitations thereto.

The transformation step in the present disclosure may be performed using gold particle bombardment.

### Advantageous Effects of Invention

The present disclosure relates to a vector system for the transformation of *Chlorella vulgaris,* a method for transforming *Chlorella vulgaris* using same, and a *Chlorella vulgaris* transformant.

### Brief Description of Drawings

FIG. 1 is a map of the vector pKA650 according to an embodiment of the present disclosure.
FIG. 2 is a photographic image elucidating that transformation using a glass bead method is low in efficiency and is poorly performed.
FIG. 3 is a photographic image of mutants that are resistance to the antibiotic as a result of transformation with a resistant gene-carrying vector system according to an embodiment of the present disclosure.
FIG. 4 is a photographic image confirming the genetic information of a mutant acquired according to an embodiment of the present disclosure.
FIG. 5 is a photographic image showing the maintenance of the mutants after passages according to an embodiment of the present disclosure.
FIG. 6 is a map of the vector pJG002 according to an embodiment of the present disclosure.
FIG. 7 is a photographic image of cells that have undergone biolistic transformation as in Example 2 and are observed to maintain resistance to the antibiotic by concentration after passages according to an embodiment of the present disclosure.
FIG. 8 is a photographic image confirming the gene and protein expression of the transformant at a band size of about 43 kDa, corresponding to that of the target protein beta-type carbonic anhydrase, as analyzed by whole protein SDS-PAGE according to an embodiment of the present disclosure.
FIG. 9 shows a MALDI-TOF spectrum confirming the band of the target protein according to an embodiment of the present disclosure.
FIG. 10 is a photographic image determining the presence or absence of the target gene as analyzed by southern blotting according to an embodiment of the present disclosure.
FIG. 11 shows photographic images comparing Sr biomineralization between the wild type and the transformant according to an embodiment of the present disclosure.
FIG. 12 is a graph showing quantitative comparison of Sr biomineralization between the wild type and the transformant, as analyzed by ICP-MS according to an embodiment of the present disclosure.

### Best Mode for Carrying out the Invention

A biomineralization vector for transformation of *Chlorella vulgaris,* comprising:
a promoter for *Coccomyxa* C-169 ribulose-1,5-bisphosphate carboxylase/oxygenase (Rubisco) small subunit 2 (rbcS2) gene;
a target protein-encoding a nucleotide sequence coding for the beta-type carbonic anhydrase of Coccomyxa subellipsoidea C-169; and
a terminator sequence of *Coccomyxa* C-169 rbcS2 gene.

### Mode for Carrying out the Invention

A better understanding of the present disclosure may be obtained through the following examples that are set forth to illustrate, but are not to be construed to limit the present disclosure.

### PREPARATION EXAMPLE 1. Plasmid Construction and Cloning

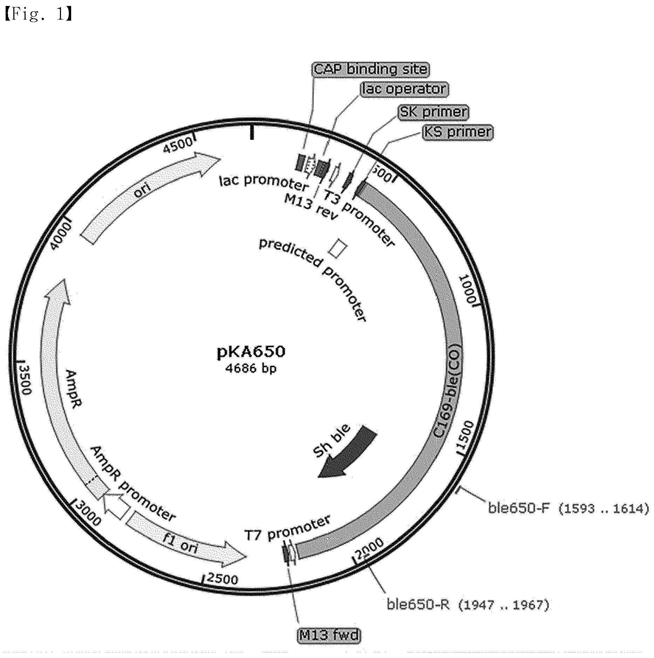

The sequence of the bleomycin resistance gene *(Sh ble*) was obtained from the genomic DNA of Streptomyces verticillus. A promoter sequence for *Coccomyxa* C-169 rbcS2 gene and a terminator sequence of for *Coccomyxa* C-169 rbcS2 gene were joined to the 5' and 3' ends of *Coccomyxa* C-169 rbcS2, respectively, to synthesize the entire sequence *Sh-ble* (GenScript, USA). The synthesized gene was treated with restriction enzymes Swa I and Kpn I and then inserted into the pSP124S vector. The obtained plasmid was named pKA650, and the vector map thereof is represented in FIG. 1.

As can be seen in FIG. 1, the vector map represents OriV for a replication origin, *Sh ble* for a gene of *Streptomyces verticillus* bleomycin with its promoter and terminator sequences, AmpR for an ampicillin resistance gene, and C-169-ble for a promoter sequence for *Coccomyxa* C-169 *rbcS2* gene at the 5' end, and a terminator sequence of *Coccomyxa* C-169 *rbcS2* gene at the 3' end.

### PREPARATION EXAMPLE 2. Transformation of Chlorella vulgaris

### 2-1 Transformation Using Glass Bead Method

A Simple and Rapid Glass Bead Transformation Method was used through glass beads. In this method, glass beads are added to cells and physical force is applied to create holes in the cell membrane through physical friction with the cell, thereby introducing a desired plasmid into the cells.

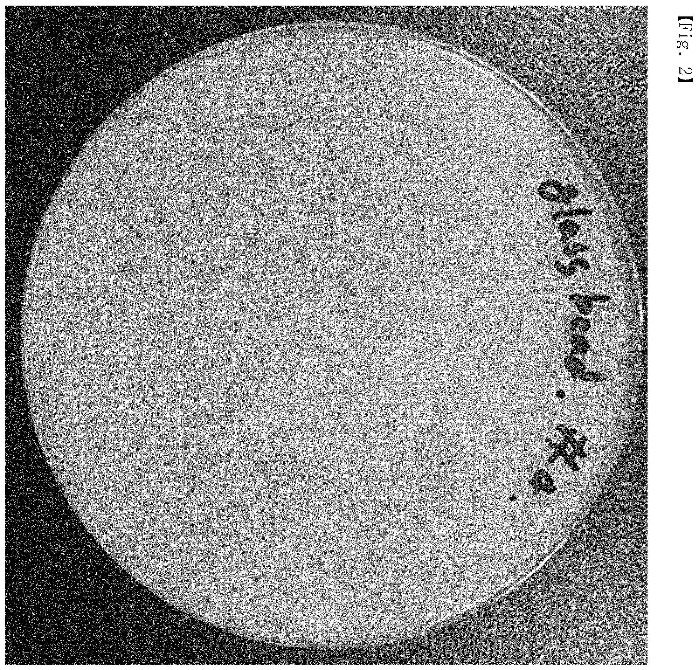

The experiment was conducted according to the conditions described in Table 1. Specifically, cells were lysed at room temperature (25°C) by vortexing with glass beads. Then, beads and supernatant were separated by gravity, and DNA was added to the supernatant. Next, recovery was carried out by slowly rotating at 37°C. After applying onto an antibiotic-containing solid medium, transformants were confirmed. The results are shown in FIG. 2.

**TABLE 1**

| **Date** | **OD_{68 6}** | **Cell number** | **Concentratio n** | **Amount of DNA** | **Vortexin g** | **Result s** |
|---|---|---|---|---|---|---|
| 16081 0 | 0.1 | 1*10⁶ cells/m 1 | 1*10⁸ cells/ml | 10 ug linearize d | 15 see | NO |
| 16081 1 | 0.3 | 1*10⁶ cells/m 1 | 1*10⁸ cells/ml | 10 ug linearize d | 10 see | NO |
| 16081 6 | 0.3 | 1*10⁷ cells/m 1 | 1*10⁹ cells/ml | 10 ug non-linearize d | 20 see | NO |

As shown in FIG. 2, it was found that the transformation was poor, with the efficiency low.

### 2-2. Transformation Using Gene Gun Method

Transformation was carried out by the plant transformation technique gold particle bombardment using a gene gun. This technique, also referred to as microprojectile acceleration or biolistics, and the gene gun is officially called microparticle bombardment. In the method, transformation is performed by coating a plasmid with micro particles. The micro particles, being very heavy relative to their size, penetrate the cells effectively. By shooting the micro particles towards the cells at high speed and surrounding them with a steel net, more particles are directed towards the cells. The DNA coated with the micro particles that enter the cells are released and can integrate into the plant's genome. For use in transformation, gold particles can be used as the micro particles.

Specifically, experiments were carried out using gold particle bombardment for transformation under various conditions shown in Table 2, below. The experiments were performed in consideration of cell stages and concentrations, shooting ranges of gold particles, target distances, and experimental environments of cells, while the vacuum and helium pressure conditions were fixed.

During the condition-establishment process, the experiments were performed on dishes with diameters of 47mm, 50mm, and 60mm in consideration with the gold particle range and pressure of the gene gun, and the appropriate Target Distance was established as Target Distance 3. Also, as the cell stage advances, the algal membrane thickens, making experiments difficult. Thus, experiments were conducted considering changes in OD values from the initial stage. Additionally, cell density was adjusted according to the diameter.

**TABLE 2**

| **Date** | **OD₆₈₆** | **Cell Density** | **Vacuum (inches Hg)** | **Target Distance** | **Helium Pressure** | **Result s** |
|---|---|---|---|---|---|---|
| 16060 3 | 0.5 | 4.0*107 per 60mm Diamete r | 29 | 3, 6, 9 | 1300 | NO |
| 16060 7 | 0.7 | 6.0*107 per 50mm Diamete r | 29 | 3 | 1300 | NO |
| 16060 7 | 0.7 | 6.0*108 per 50mm Diamete r | 29 | 6 | 1300 | NO |
| 16060 7 | 0.7 | 6.0*109 per 50mm Diamete r | 29 | 9 | 1300 | NO |
| 16061 3 | 0.07 4 | 3.6*106 per 50mm Diamete r | 29 | 3, 6, 9 | 1300 | NO |
| 16061 4 | 0.1 | 3.6*106 per 50mm Diamete r | 29 | 3.6.9 | 1300 | NO |
| 16061 5 | 0.2 | 3.5*106 per 50mm Diamete r | 29 | 3.6.9 | 1300 | NO |
| 16061 6 | 0.3 | 4.0*106 per 50mm Diamete r | 29 | 3.6.9 | 1300 | NO |
| 16061 7 | 0.6 | 3.6*106 per 50mm Diamete r | 29 | 3.6.9 | 1300 | NO |
| 16062 9 | 0.1 | 4.8*107 per 60mm Diamete r | 29 | 3.6.9 | 1300 | NO |
| 16063 0 | 0.2 | 4.8*107 per 60mm Diamete r | 29 | 3.6.9 | 1300 | NO |
| 16070 1 | 0.3 | 4.8*107 per 60mm Diamete r | 29 | 3.6.9 | 1300 | NO |
| **16070 4** | **0.6** | **4.8*107 per 60mm Diamete r** | **29** | **3.6.9** | **1300** | **YES** |
| 16071 1 | 0.1 | 1.0*107 per 47mm Membran e | 29 | 3 | 1300 | NO |
| 16071 2 | 0.2 | 1.0*107 per 47mm Membran e | 29 | 3 | 1300 | NO |
| 16071 3 | 0.3 | 1.0*107 per 47mm Membran e | 29 | 3 | 1300 | NO |
| 16071 4 | 0.6 | 1.0*107 per 47mm Membran e | 29 | 3 | 1300 | NO |
| 16081 5 | 0.07 | 2.0*107 per 47mm Membran e | 29 | 3 | 1300 | - |
| 16081 6 | 0.1 | 2.0*107 per 47mm Membran e | 29 | 3 | 1300 | - |
| 16081 8 | 0.3 | 2.0*107 per 47mm Membran e | 29 | 3 | 1300 | - |

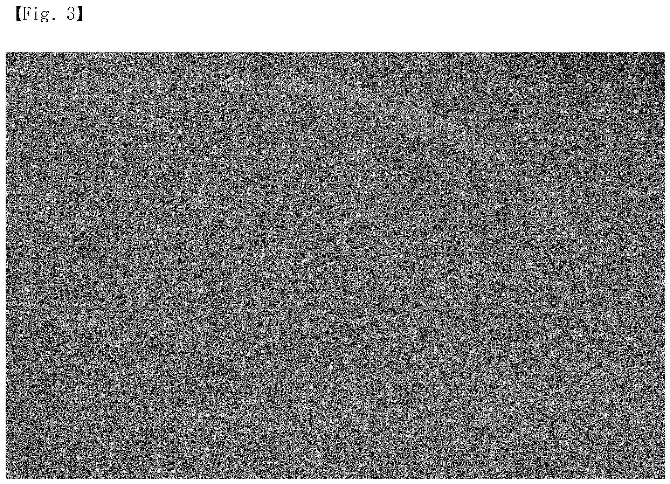

As shown in Table 2 and FIG. 3, in the case of failure, growth did not occur on solid medium containing antibiotics, while success resulted in colony formation. Under the condition of 160704, the operation of the promoter (mutant) was confirmed. Then, the target protein (carbonic anhydrase) was introduced under the same conditions, forming mutants.

### EXPERIMENTAL EXAMPLE 1. Genetic Information Confirmation

To confirm the genetic information, genomic DNA was secured and the introduced gene was confirmed through PCR and DNA sequencing. Specifically, for use in PCR, the primer set of Table 3 was constructed. In the presence of the primer set, PCR started by pre-denaturation at 98°C for 8 minutes and was continued with 30 cycles of 98°C for 1 minute, 53.5°C for 30 seconds, and 72°C for 1 minute, followed by 72°C for 7 minutes. The PCR product was analyzed by sequencing, and the results are summarized in Table 3.

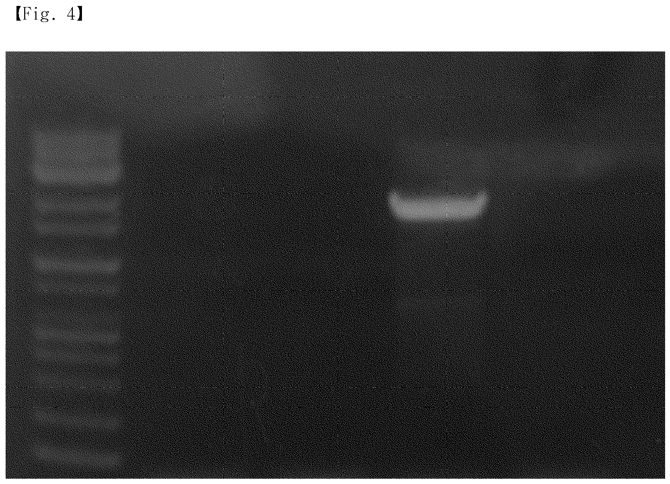

Additionally, to confirm the entire sequence *Sh-ble,* obtained by joining the promoter sequence of the *Coccomyxa* C-169 rbcS2 gene at the 5' end and the terminator sequence at the 3' end to the bleomycin resistance gene, PCR was performed using the M13 primer set. Starting at 98°C for 8 minutes for pre-denaturation, PCR was carried out with 30 cycles of 98°C for 1 minute, 54°C for 40 seconds, 72°C for 2 minutes 30 seconds, followed by extension at 72°C for 7 minutes, and the target DNA band was confirmed. The results are shown in FIG. 4.

**TABLE 3**

| SEQ ID NO: | Name | Sequence listing (5'→ 3') | Note |
|---|---|---|---|
| 5 | Forward primer | ATGGCCAAGTTGACCAGT | |
| 6 | Reverse primer | TCAGTCCTGCTCCTCGGCCA | |

As can be seen in FIG. 4, a band was detected at about 2kb, the total size of the target gene. DNA gene analysis was conducted to determine whether the band matched the target gene, and the results are shown in Table 4.

**TABLE 4**

| | |
|---|---|
| sh-ble TF5ble1 | -CGGCAATGACTGATTACGCCAGCTTGGTACCGAGCTCGGATCCACTAGTAACGGCCGCCA |
| sh-ble TF5ble1 | --------------------ATGGCCAAGTTGACCAGTGCCGTTCCGGTGCTCACCGCGC GTGTGCTGGAATTCGCCCTTATGGCCAAGTTGACCAGTGCCGTTCCGGTGCTCACCGCGC **************************************** |
| sh-ble TF5ble1 | GCGACGTCGCCGGAGCGGTCGAGTTCTGGACCGACCGGCTCGGGTTCTCCCGGGACTTCG GCGACGTCGCCGGAGCGGTCGAGTTCTGGACCGACCGGCTCGGGTTCTCCCGGGACTTCG ************************************************************ |
| sh-ble TF5ble1 | TGGAGGACGACTTCGCCGGTGTGGTCCGGGACGACGTGACCCTGTTCATCAGCGCGGTCC TGGAGGACGACTTCGCCGGTGTGGTCCGGGACGACGTGACCCTGTTCATCAGCGCGGTCC ************************************************************ |
| sh-ble TF5ble1 | AGGACCAGGTGGTGCCGGACAACACCCTGGCCTGGGTGTGGGTGCGCGGCCTGGACGAGC AGGACCAGGTGGTGCCGGACAACACCCTGGCCTGGGTGTGGGTGCGCGGCCTGGACGAGC ************************************************************ |
| sh-ble TF5ble1 | TGTACGCCGAGTGGTCGGAGGTCGTGTCCACGAACTTCCGGGACGCCTCCGGGCCGGCCA TGTACGCCGAGTGGTCGGAGGTCGTGTCCACGAACTTCCGGGACGCCTCCGGCCGGCCA ************************************************************ |
| sh-ble TF5ble1 | TGACCGAGATCGGCGAGCAGCCGTGGGGGCGGGAGTTCGCCCTGCGCGACCCGGCCGGCA TGACCGAGATCGGCGAGCAGCCGTGGGGGCGGGAGTTCGCCCTGCGCGACCCGGCCGGCA *********************************************************** |
| sh-ble TF5ble1 | ACTGCGTGCACTTCGTGGCCGAGGAGCAGGACTGA ACTGCGTGCACTTCGTGGCCGAGGAGCAGGACTGA *********************************** |

As can be seen in Table 4, a complete match was confirmed when comparing the DNA nucleotide sequence of the target gene, Sh-ble, with the acquired genetic information, as analyzed by CLUSTAL 2.1 multiple sequence alignment.

### EXPERIMENTAL EXAMPLE 2. Maintenance of Mutants

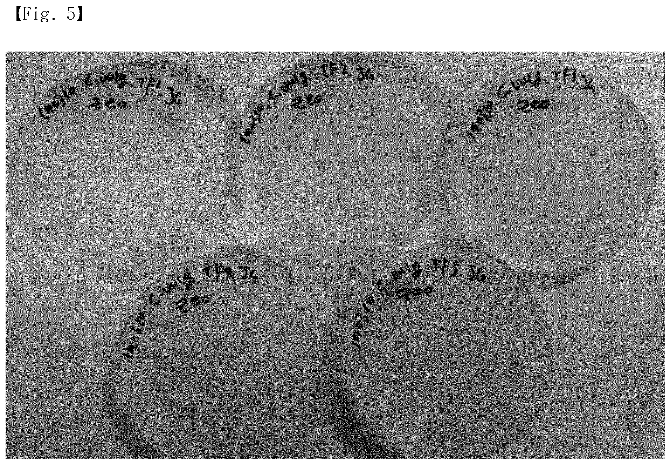

To confirm the maintenance of mutants, passages were performed, and the results are shown in FIG. 5. As can be seen in FIG. 5, mutants were maintained by passages.

### EXPERIMENTAL EXAMPLE 3. Biomineralization

### Example 1: Plasmid Construction and Cloning

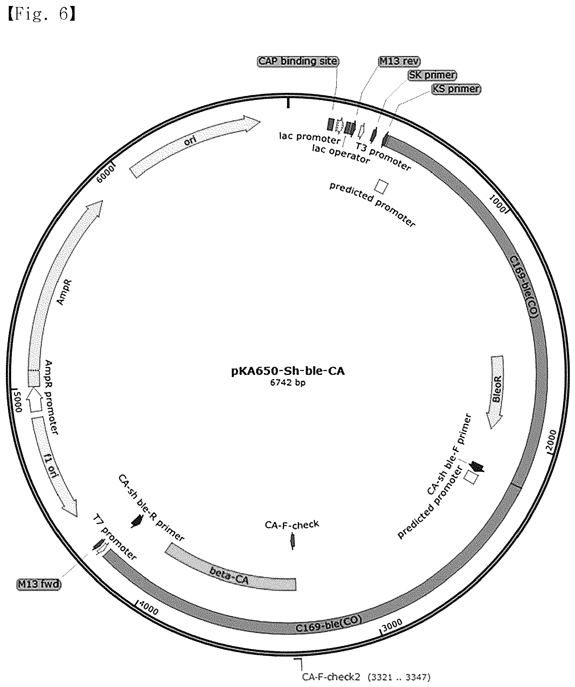

pKA650 vector was constructed for expressing and cloning a gene for a target protein, with resistance to zeocin given thereto. In this regard, the bleomycin resistance gene sequence (*Sh ble*) was obtained from the genomic DNA of *Streptomyces verticillus* (*Sh ble*). An overall sequence was synthesized by linking the promoter and terminator sequences of the *Coccomyxa* C-169 rbcS2 (ribulose-1,5-bisphosphate carboxylase/oxygenase small subunit) gene to the 5' and 3' ends of *Sh ble,* respectively. The synthesized sequence was named pKA650. In the synthesized pKA650, the intermediate gene *BleoR* of the entire sequence *Sh-ble* was substituted for C-169 *beta-type carbonic anhydrase,* after which repeated promoter region was inserted with the aid of *Kpn*I and *Apa*I. The cloned plasmid is depicted in FIG. 6 and was named pJG002.

### Example 2: Chlorella vulgaris Transformation

The method of transformation using the pJG002 vector is same as that using the pKA650 vector.

Specifically, competent cells were created. In this regard, 300 mL of sterile MBM medium (KNO₃ 2.5mM, MgSO₄7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L) was prepared in a 500 mL flask and inoculated with *Chlorella vulgaris,* followed by incubation at 23°C and 100 rpm. When OD₆₈₆ reached 0.5-0.6, *Chlorella vulgaris* was collected by centrifugation and counted.

After being counted, *Chlorella vulgaris* was prepared at a density of 4.8*10⁷ cells. For gold particle bombardment, the introduced pKA650 was linearized using the restriction enzyme KpnI. A retaining cap, a brass adjustable nest, a microcarrier holder, a stopping screen, and a macrocarrier were prepared as being sterilized, and a rupture disk was washed with 70% isopropanol. While drying the rupture disk, a plasmid and gold particle mixture was prepared. For 20 bombardments, 12 mg of gold particles was put in a microfuge tube, and 1 ml of 70% ethanol was added thereto. The tube was vortexed at maximum speed for 5 minutes and briefly spun down for 5 seconds, followed by decanting the supernatant.

The cycle of washing gold particles with 1 ml of distilled water, vortexing for 1 minutes, leaving the gold particles for 1 minutes, spinning down for 5 seconds, and decanting the supernatant was repeated three times. Then, the addition of 205 µL of 50% glycerol was followed by vortexing for 5 minutes to suspend the pellet. Vortexing was continued at speed 2-3. One hundred µL of gold particles was transferred to a new microfuge tube, and 10 µL of DNA (0.5-20 pg/pL), 100 µL of 2.5M CaCl₂, and 40 µL of 0.1 M spermidine were added sequentially while being mixed by pipetting. After mixing, the mixture was vortexed continued for 2 minutes, left for 1 minute, and spun down for 2 seconds. After removal of the supernatant, the pellet was added with 300 µL of 70% ethanol and left for 1 minute. The supernatant was decanted after which 300 µL of 100% ethanol was added and left for 1 minute. Again, the supernatant was removed, and 110 µL of 100% ethanol was added and continuously vortex to suspend the pellet.

Once prepared, the Gold Particle Mix was placed in an amount of 11 µL in the center of the microcarrier and dried for 5-10 minutes. Meanwhile, the prepared cells were evenly spread on the plate with a diameter of 60 mm. The stopping screen was mounted on the brass adjustable nest, followed by loading the dried microcarrier thereon. After fixation with a holder, the gene gun instrument was turned on and preheated for 5 minutes. When preheating was completed, the rupture disk was loaded into a retaining cap and inserted into the gun part by screwing. Then, the microcarrier-assembled brass adjustable nest was inserted and the plate having the cells spread thereon was mounted at target distance 3 before closing the door. Helium gas was introduced and the vacuum button was pressed. When the vacuum and the helium pressure reached 29 (inches Hg) and 1300 psi, respectively, the fire button was pressed to increase the pressure to 1100 psi at which gold particles are shot. After bombardment, the plate was incubated at 23°C for recovery.

### EXAMPLE 3: Confirmation of Chlorella vulgaris Transformant

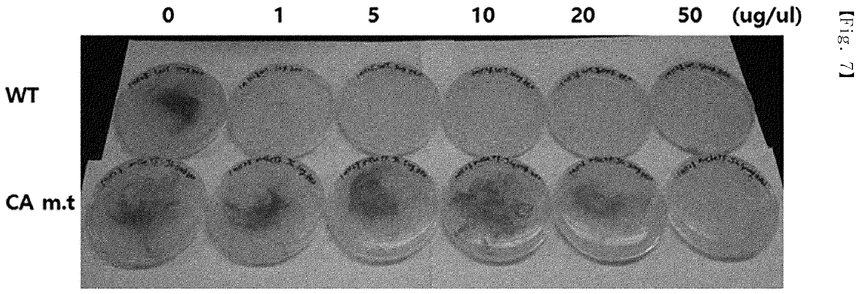

The transformation was performed biolistically as in Example 2, and after passages, the transformants were tested for maintaining antibiotic resistance at various concentrations. The results are shown in FIG. 7. A total of 42 transformants were obtained. They were continuously passaged in the medium of Example 2 while being exposed to light.

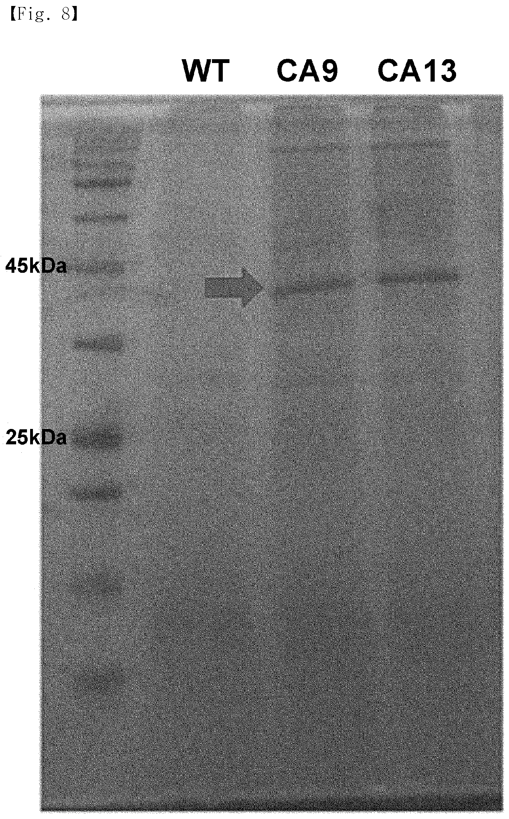

The genetic and protein expression of the transformants was examined. To this end, the target protein, beta-type carbonic anhydrase was detected at a band size of approximately 43 kDa as analyzed by whole protein SDS-PAGE. The results are shown in FIG. 8.

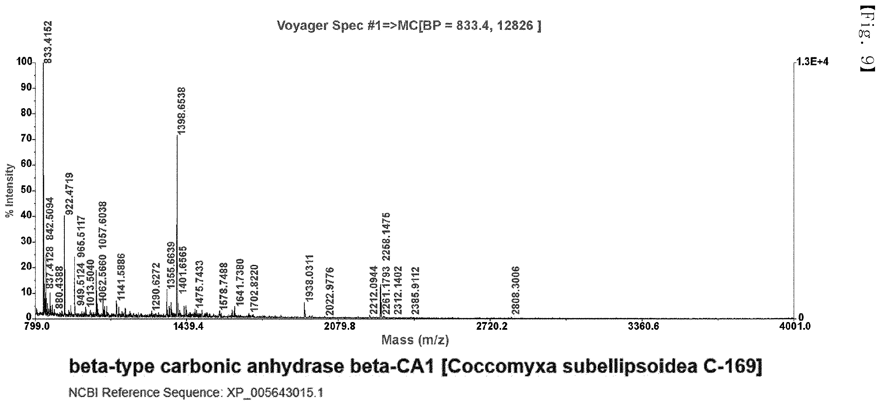

The band for the target protein was determined by MALDI-TOF, and the results are depicted in FIG. 9.

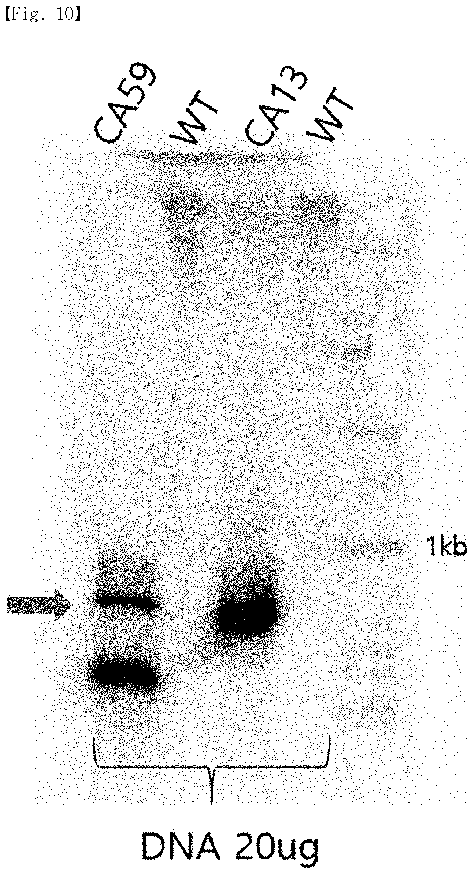

In the present disclosure, the transformants were found to express the target protein and maintain antibiotic resistance over passages. For the genetic aspect, the presence or absence of the target gene was determined by southern blotting, and the results are depicted in FIG. 10.

As seen in FIG. 10, a DNA band was detected at the size corresponding to the target gene, and PCR and DNA sequencing also demonstrated the target gene.

### EXAMPLE 4: Sr Biomineralization

The function of the transformant was to provide an increase in Sr biomineralization capacity. This was confirmed by visualizing crystal formation under a microscope with equal amounts. Initially, the Sr biomineralization capability of the wild-type (WT) *Chlorella vulgaris* was confirmed.

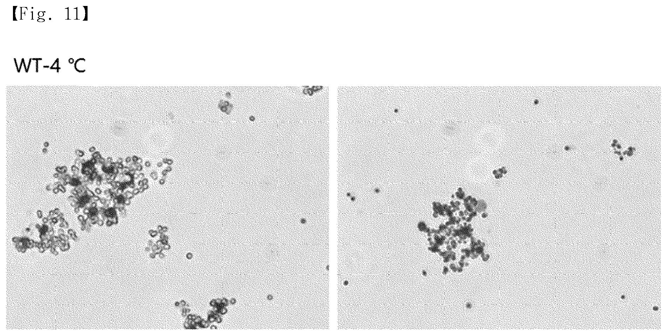

In brief, *Chlorella vulgaris* was cultured under light conditions until OD0.6. After cultivation, the microalgae were collected at 25°C and 4000 rpm for 15 minutes in a sterile condition. They were then washed three times with 3mM NaHCO₃. Subsequently, 3 mM NaHCO₃ was added with 200 ppm Sr, mixed with 1*10⁷ *Chlorella vulgaris,* and incubated at 4°C for 4 hours or longer. The cells were then stained with 0.004% sodium rhodizonate and observed under a microscope, with the results shown in FIG. 11.

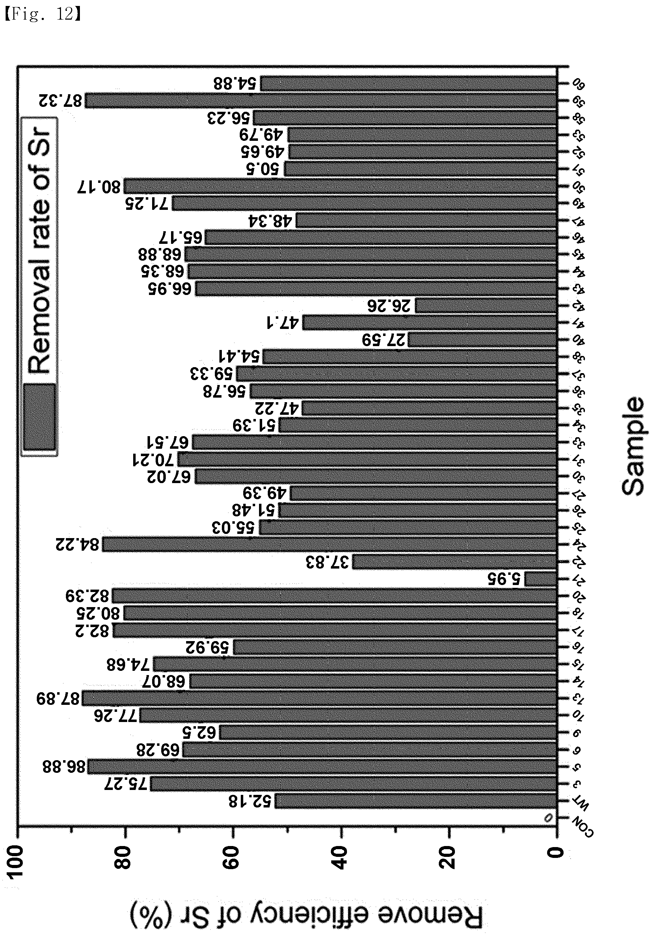

As can be seen in FIG. 11, the crystallization of WT showed a lower amount of biomineralized crystals compared to the transformant. For quantitative comparison, ICP-MS analysis was performed, and the results are shown in FIG. 12 and Table 5.

**TABLE 5**

| | Remain metal ion rate (%) | RSD(%) : Relative Standard Deviation |
|---|---|---|
| Control (200 mM LiCl2) | 100 | 0.56 |
| + algae | 45.27667 | 0.42 |

As seen in FIG. 12 and Table 5, the transformants exhibited up to 160 % increased biomineralized crystals compared to the wild type.

### Industrial Applicability

The present disclosure relates to a vector system for the transformation of *Chlorella vulgaris,* a method for transforming *Chlorella vulgaris* using same, and a *Chlorella vulgaris* transformant.

## Claims

1. A biomineralization vector for transformation of *Chlorella vulgaris,* comprising:
a promoter for *Coccomyxa* C-169 ribulose-1,5-bisphosphate carboxylase/oxygenase (Rubisco) small subunit 2 (rbcS2) gene;
a nucleotide sequence coding for beta-type carbonic anhydrase of Coccomyxa subellipsoidea C-169; and
a terminator sequence of *Coccomyxa* C-169 rbcS2 gene.

2. The biomineralization vector of claim 1, wherein the promoter comprises the nucleotide sequence of SEQ ID NO: 2.

3. The biomineralization vector of claim 1, wherein the target protein-encoding nucleotide sequence is operatively linked to the promoter.

4. The biomineralization vector of claim 1, wherein the terminator sequence comprises the nucleotide sequence of SEQ ID NO: 3.

5. The biomineralization vector of claim 1, wherein the vector system further comprises a gene resistant to an antibiotic as a selectable marker.

6. The biomineralization vector of claim 5, wherein the antibiotic is at least one selected from the group consisting of spectinomycin, paromomycin, ampicillin, zeocin, and bleomycin.

7. The biomineralization vector of claim 1, wherein the vector system further comprises a gene coding for a reporter molecule.

8. The biomineralization vector of claim 7, wherein the reporter molecule is at least one selected from the group consisting of a growth-promoting protein, a fluorescent protein, and a hydrolase.

9. The biomineralization vector of claim 7, wherein the fluorescent protein is luciferase.

10. The biomineralization vector of claim 7, wherein the hydrolase is β-glucuronidase.

11. The biomineralization vector of claim 1, wherein the vector is designed to use gold particles bombardment for transformation of microalgae.

12. *Chlorella vulgaris* transformed with the vector of any one of claims 1 to 11.
